# EUROPEAN PATENT SPECIFICATION

(11) **EP 2 371 819 B1**
(45) Date of publication and mention of the grant of the patent: **10.01.2018**
(21) Application number: 09830644.2
(22) Date of filing: 02.03.2009
(51) Int. Cl.: C07D 215/18, C07D 215/14, A61K 31/47, A61P 31/06

(54) **NEW QUINOLINE DERIVATIVES, A METHOD FOR THE PRODUCTION AND THE USE THEREOF FOR TREATING MICROBACTERIAL INFECTIONS AND A PHARMACEUTICAL COMPOSITION BASED ON SAID DERIVATIVES**
NEUE CHINOLINDERIVATE, HERSTELLUNGSVERFAHREN UND DEREN VERWENDUNG ZUR BEHANDLUNG MIKROBAKTERIELLER INFEKTIONEN, UND EINE AUF DIESEN DERIVATEN BASIERENDE PHARMAZEUTISCHE ZUSAMMENSETZUNG
NOUVEAUX DERIVES DE QUINOLEINE, PROCEDE DE FABRICATION ASSOCIE, METHODE D'UTILISATION DE CES DERIVES DANS LE TRAITEMENT DES INFECTIONS MYCOBACTERIENNES ET COMPOSITION PHARMACEUTIQUE LES CONTENANT

(30) Priority: 02.12.2008 RU 2008147273
(43) Date of publication of application: 05.10.2011
(73) Proprietor: Aktsionernoe Obschestvo "Pharm-Sintez", Moskow, 111024 (RU)
(72) Inventor: ZHAROV, Aleksey Nikolaevich, Moscow 117570 (RU); SAVOSTYANOV, Sergey Vladimirovich, Moscow 129347 (RU); TAGACHENKOV, Andrey Alekseevich, Moscow 109472 (RU); IVANOV, Andrey Sergeevich, Moscow 105066 (RU); FEDOROV, Vladimir Egorovich, Moscow 125008 (RU); PERMINOV, Sergey Vladimirovich, Moscow 109444 (RU)
(74) Representative: Held, Stephan
(86) International application number: PCT/RU2009/000097
(87) International publication number: WO 2010/064951

(56) References cited:
- WO-A1-2004/011436
- WO-A1-2005/117875
- WO-A1-2006/131519
- CA-A1- 2 529 265
- RU-A- 2006 124 843
- US-A1- 2005 148 581
- S. PETIT ET AL: "Absolute configuration and structural features of R207910, a novel anti-tuberculosis agent", JOURNAL OF MOLECULAR STRUCTURE, vol. 837, no. 1-3, 1 June 2007 (2007-06-01), pages 252-256, XP55024315, ISSN: 0022-2860, DOI: 10.1016/j.molstruc.2006.11.001
- A. ARJONA ET AL: "TMC-207", DRUGS OF THE FUTURE, vol. 33, no. 12, 1 January 2008 (2008-01-01), page 1018, XP55024323, ISSN: 0377-8282, DOI: 10.1358/dof.2008.33.12.1291333
- R L Madan: "Organic Chemistry" In: "Organic Chemistry", 1 January 2013 (2013-01-01), Tata McGraw-Hill Education, XP55332421, ISBN: 978-1-259-00618-0 pages 643-644,

## Description

The invention is in the class of the biologically active compounds, in particular, compounds having activity against mycobacteria. The invention relates, as well, to the process of their production, to said compounds for use in the treatment of tuberculosis and other infectious diseases caused by mycobacteria, and to the pharmaceutical composition on their basis.

The etiological agent of the tuberculosis is the *Mycobacterium tuberculosis.* According to the World Health Organization, the tuberculosis develops every year in more than 8 million people. The approaches to the treatment of this disease, used as of today, are based on combining several preparations, such as isoniazid, rifampicine, pyrazinamide. In the event of the tuberculosis infected patients subject to the multidrug resistance, the therapy additionally uses such antimicrobial preparations as the ethambutol, streptomycin, kanamycin, amikacin, ethionamide, cycloserine, ciprofloxacin, ofloxacin, etc. Taking into account the increasing rate of development of resistant forms of tuberculosis in the world and the low efficiency of the preparations used today to deal with such forms of tuberculosis, the search for a new original antituberculous drug is an urgent challenge to the today's medicine.

The long and well known drug is quinine (formula **I**): 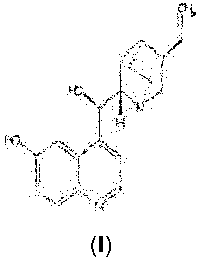 which is a well known natural antibiotic with antimalarial effect.. It was first extracted from the cinchona bark in 1817; its use to treat the malaria was mentioned in the 17th century documents. Despite the well studied pharmacological properties of the quinine itself and its closest analogs, the aminoquinolinic pharmacophore has a yet undiscovered potential as for the treatment of other bacterial infections. Quinine is first of a whole group of drugs used to treat the bacterial infections. Another example of the drugs of the aminoquinolinic line is the mefloquine, which is, just as the quinine, used to treat the malaria. Drugs of the Futire, vol. 33, no. 12, 1 January 2008, p. 1018 disclose bedauiline, an another drug of this line. The purpose of this invention consisted in finding the new derivatives of the aminoquinoline that would have a high antimycobacterial activity, including those resistant to the traditional preparations, such as the rifampicine and isoniazide.
This has resulted in the synthesis of the line of the aminoquinolines which are structural analogs of the quinine; their activity in relation to the mycobacterial infections was studied from the viewpoint of creation of a new antituberculous preparation on their basis, in particular, on the basis of compounds of the general formula **II**, including their pharmaceutically acceptable acid- or base-additive salts, stereochemical isomeric forms. The subject matter of the application is described in the appended claims and relates to compounds of the general formula (II) and the use thereof. Compound of formula (II) is presented with the following structure. 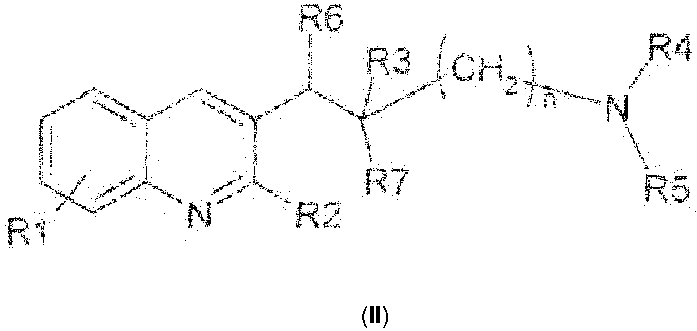 where
- R¹: means hydrogen, halogen, haloalkyl, dihaloalkyl, trihaloalkyl, cyano,
- R²: means halogen,
- R³: means hydroxy, mercapto, alkylthio, [amino(imino)methyl]thio, alkyldithio, difluormethyloxy,
- R⁴,: R⁵ mean, independently, hydrogen, alkyl, benzyl; or together, including the atom of nitrogen, they mean morpholinyl, piperidinyl, pyrrolidinyl, imidazolyl, triazinyl, tetrahydroazepinyl, piperazinyl, *N*'-alkylpiperazinyl, homopiperazinyl, *N'-*alkylhomopiperazinyl, pyrrolidinyl,
- R⁶: means aryl, heteroaryl,
- R⁷: means aryl, heteroaryl,
n = 1-4,
and their stereoisomers and mixes in any proportion, pharmaceutically acceptable acidic or basic additive salts, including the solvates.

Alkyl means a straight or branched saturated hydrocarbonic radical having 1 to 6 atoms of carbon.

Aryl means a homocycle selected from a group that includes phenyl, naphthyl, acenaphthyl, tetrahydronaphtyl, of which any one may be substituted with 1, 2, or 3 substituting groups, and any substituting group is independently selected from the groups including hydroxy, halogen, cyano, mono- or di- alkylamino, alkyl, haloalkyl, alkyloxy, haloalkoxy, carboxyl, alkyloxycarbonyl, aminocarbonyl, morpholine and mono or dialkylaminocarbonyl.

Heteroaryl means a monocyclic heterocyclic ring, selected from a group including *N*'-phenoxypiperidinyl, pyrrolyl, imidazolyl, furanyl, thienyl, oxazolyl, isoxazolyl, thiazolyl, isothiazolyl, pyridinyl, pyrimidinyl, pyrasinyl, and pyridazinyl, or a bicyclic heterocyclic ring, selected from a group including the quinolinyl, quinoxalinyl, indolyl, benzimidazolyl, benzoxazolyl, benzisoxazolyl, benzothiazolyl, benzithiazolyl, benzothienyl, 2,3-dihydrobenzo[1,4]dioxinyl or benzo[1.3]dioxolyl, where in any monocyclic or bicyclic heterocyclic ring an atom of carbon may be substituted with 1, 2, or 3 substituting groups, selected from the group including halogen, hydroxy, alkyl, or alkyloxy.

The pharmaceutically acceptable additive salts with acids are characterized by that they contain the therapeutically active nontoxic additive saline forms capable to form the compounds of the formula **II**. The above additive salts with acids may be obtained by treating a compound in form of the bases represented by formula **II** with the appropriate acids, for example, the inorganic acids, for example, the hydrohalogenic acid, in particular, the hydrochloric acid, hydrobromic acid, sulfuric acid, nitric acid, and phosphoric acid; with the organic acids, for example, the acetic acid, hydroxyacetic acid, propionic acid, lactic acid, pyroracemic acid, oxalic acid, malonic acid, maleic acid, fumaric acid, malic acid, tartaric acid, citric acid, methanesulfonic acid, ethanesulfonic acid, benzolsulfonic acid, n-toluolsulfonic acid, cyclamic acid, salicylic acid, n-aminosalicylic acid.

The compounds represented by formula **II**, containing the acid protons may also be converted into their therapeutically active additive salt forms with bases by treating them with the appropriate organic and inorganic bases; the appropriate salt forms with bases include, for example, the salts of ammonium, the salts of alkaline and alkaline earth metals, in particular, the salts of lithium, sodium, potassium, magnesium, and calcium, the salts with organic bases, for example, the salts of benzathine, *N*-methyl-(*D*)-glucamine, hydramine, and salts with amino acids, for example, with arginine and lysine.

Further is described: The above additive saline forms with acids or bases may be transformed into free forms by treating them with an appropriate base or acid.

The term "additive salts" used for this invention includes the solvates capable to form the compounds of the formula **II**. These solvates are, for example, the hydrates and alcoholates.

The term "stereochemical isomerous forms" used here covers all the possible isomerous forms that the compounds of the formula **II** may have. Unless specially mentioned or indicated differently, the chemical notations of the compounds cover the mix of all the possible stereochemical isomerous forms and the said mixes contain all the diastereoisomers and enantiomers of the main molecular structure. More precisely, the steric centers may have the (*R*)- or (*S*)-configurations, the substituting group at the bivalent cyclic (partly) saturated radicals may have either the cis- or trans- configuration.

The stereochemical isomeric forms of the compounds of formula **II** must, obviously, be covered by the field of this invention.

It was revealed that the compounds as claimed are for use in the treatment of mycobacterial diseases, in particular, the ones caused by pathogenic mycobacteria, such as *Mycobacterium tuberculosis.*

The invention also includes the pharmaceutical composition, including the active substance, the pharmaceutically acceptable carrier and auxiliary substances; for the active substance it contains an efficient quantity of the compounds as claimed.

To prepare the pharmaceutical compositions as claimed, an efficient quantity of an active compound, not necessarily in form of an additive salt, is combined into a fine mixture with a pharmaceutically acceptable carrier; the forms may widely vary depending upon the desired type of preparation to administer. These pharmaceutical compositions are desirable in form of a standard pharmaceutical form, in particular, for the oral administration or parenteral injection. For example, when preparing the compositions as a pharmaceutical form for oral administration, any common pharmaceutical medium may be used, such as, for example, water, glycols, oil, alcohols, etc.; in case of the liquid preparations for oral administration, these are suspensions, syrups, elixirs, emulsions, and solutions; in case of the pulvis, pills, capsules, and tablets, these are the solid media, such as starches, sugars, kaolin, diluents, lubricants, cohesive, disintegrating agents, etc. Due to the easy administration, the tablets and capsules are the preferable standard pharmaceutical forms for oral administration; in their case the solid pharmaceutically acceptable auxiliary substances are explicitly used. In the parenteral compositions the carrier, at least most of it, is the sterile water, together with other additions, for example, to improve the solubility. For example, in the solutions for injections, the carrier may include a salt solution, a glucose solution, or a mixture of a salt solution and a glucose solution. The suspensions for injections may use the appropriate liquid substances, suspending agents, etc. These include also the solid form preparations, intended to be transformed into liquid preparations shortly before administering.

Depending upon of the way of administration, a pharmaceutical composition will, preferably, contain 0.05% to 99% wt. (more preferably 0.1% to 70% wt.) of the active ingredient and 1% to 99.95% wt. (more preferably 30% to 99.9% wt.) of the pharmaceutically acceptable carriers (all the percents are given taking into account the whole composition).

A pharmaceutical composition may additionally contain various other known substances, for example, a lubricant, a stabilizing agent, a buffer agent, an emulsifying agent, a viscosity regulating agent, a surface-active agent, a preservative, a fragrant, or a colorant. It is especially convenient to obtain the above pharmaceutical compositions in form of a standard pharmaceutical form, for easier administration and more regular dosage. The term "standard pharmaceutical form" used here relates to the physically discrete units used as unified doses; each unit contains a set quantity of the active ingredient calculated so as to create the desired therapeutical effect, combined with the necessary pharmaceutical carrier. The examples of such standard pharmaceutical forms are the tablets (including those notched and coated), capsules, pills, sachets with pulvis, starch capsules, suppositories, solutions, or suspensions for injections, etc., and their multiple forms. The daily dose of the claimed compound will, of course, vary depending upon the used compound, the way of excretion, the desired treatment, and the specific mycobacterial disease. However, as a rule, the satisfactory results will be reached when the claimed compound is administered in the daily dose not exceeding 1 g, for example, within the limits 10 to 50 mg/kg-BM.

Further is described the use of the compound of the formula **II**, its pharmaceutically acceptable additive salt with acids and bases, its stereochemically isomeric forms, its tautomeric forms, to produce the drugs to treat the mycobacterial disease.

Another claim is the process of production of the compounds of the general formula **II**, consisting in the interaction of the 2-arylmethylquinolines and appropriate arylketones under the effect of the metalizing agents. Here the metalizing agents are usually the amides of metals, such as lithium diisopropylamide , lithium diethylamide , lithium 2,2,6,6,-tetramethylpiperidide , etc.

Accordingly, the present invention relates to the compounds of the general formula **II** for use in the treatment of infections induced by the mycobacteria. Further, the present invention relates to the claimed compounds for use in the treatment of tuberculosis.

### Overview of synthesis

The compounds claimed in formula **II** may be obtained from the intermediate compound of formula **III** and the intermediate compound of formula **IV** using a metalizing reagent, for example, the lithium diisopropylamide obtained *in situ* from the butyllithium and diisopropylamine in an appropriate solvent, preferably of the ether type, such as, for example, the diethyl ether, diisopropyl ether, methyltertbutyl ether, or their mixture, or mixture with other solvents and cosolvents (see Scheme 2). The definitions of all the radicals are as in formula **II**. The rate of reaction may be increased by agitating. The reaction may be easily realized within the range of temperatures -70 to +20°C, preferably within the range -70 to -20°C. Scheme 2

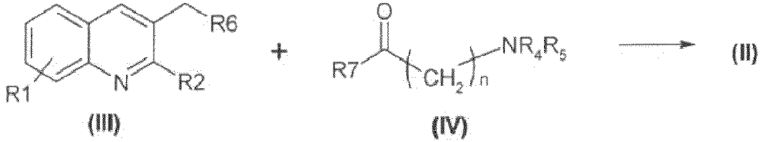

The mixture of the optical isomers resulting from the reaction is separated using the known methods such as chromatography or crystallization of the diastereoisomeric salts. The initial and intermediate compounds of the formulas **III** and **IV** are either commercially available or obtainable using the well known usual methods [D. Mabire, et al., J. Med. Chem., 2005, 48, 2134-2153]. For example, the intermediate compounds of the formula **II** are obtainable using the process shown in Scheme 3. 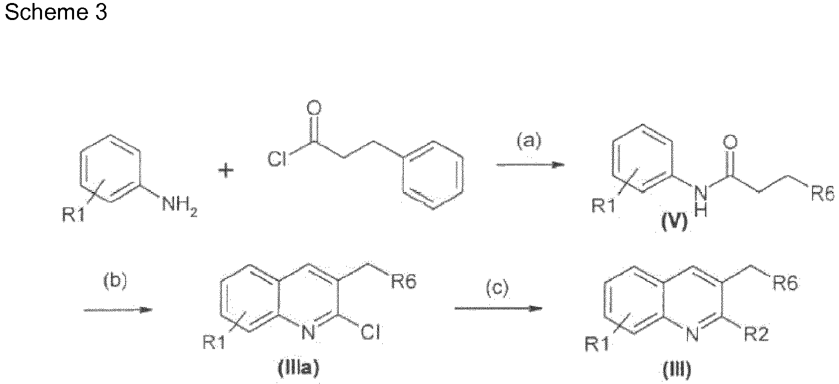 where the designations of all the radicals are as in formula **II**. The synthesis under the scheme 3 includes the stage (a) where the substituted arylamine interacts with an appropriate acylchloride, such as phenylpropionylchloride, 3-fluobenzenepropionylchloride, or n-chlorobenzenepropionylchloride, in presence of an appropriate base, such as triethylamine, and appropriate reaction inert solvent, such as methylenedichloride or ethylenedichloride. The reaction may be easily realized within the range between the room temperature and boiling temperature. At the next stage (b) the adduct obtained at the stage (a) reacts with the phosphorous oxychloride (POCl₃) in presence of the *N,N*-dimethylformamide (Wilsmeyer-Haack formylation with further ring formation). The reaction may be easily realized within the range between the room temperature and boiling temperature. At the next stage (c) the chlorine in the second position of the quinoline group is exchanged against the other groups of R², via the reaction of the intermediate compound resulting from the stage (b), with the relevant nucleophilic reagents.
Obviously, in the previous and subsequent reactions, the reaction products may be extracted from the reaction medium, and, if necessary, purified using the methods known in this field, such as the extraction, crystallization, and chromatography. Besides, obviously, the reaction products for which more than one optical isomer may exist, can be extracted from the mixture using the known methods, such as the preparative chiral high pressure liquid chromatography.

The compounds of the formula **IV** are either commercially available or obtainable using the generally known methods of synthesis. The examples below explain the claim without limitation.

### Examples of synthesis of the claimed compounds

### A. Obtaining intermediate compounds (general method).

### Case 1

### Obtaining intermediate compound Va

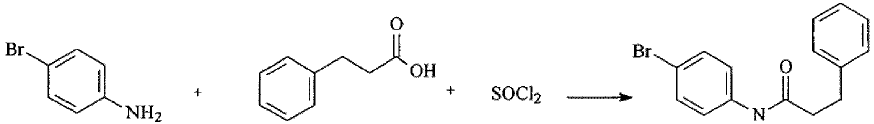

To a boiling mixture of 230 grams of the hydrocinnamic acid and 270 grams of 4-bromoaniline in 1 liter of toluol 160 ml of thionyl chloride are added by drops; then 1 liter of water and 50 ml of aqueous ammonia are added by drops. The sediment after cooling is filtered and dried. Yield:
405 grams of the intermediate compound **Va.**

### Case 2

### Obtaining intermediate compound IIIa

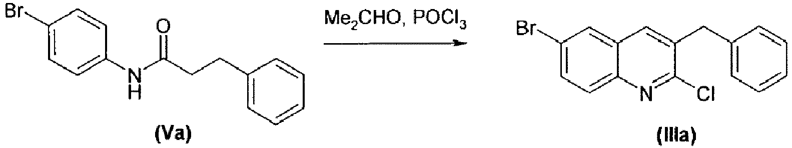

To the cooled down to 10°C mixture of 84 grams of dimethyl formamide and 200 grams of the intermediate compound **Va** in 600 ml of chloroform, 400 ml of phosphorous oxychloride are added by drops; then the mixture is boiled during the night. The mixture is then cooled and poured out into 2 kg of ice; the organic layer is flushed with water, dried above MgSO₄, and filtered; the solvent is vacuum distilled. The residue is crystallized from the methanol. Yield: 163 grams of the intermediate compound **IIIa**.

### Case 3

### Obtaining compound IVa

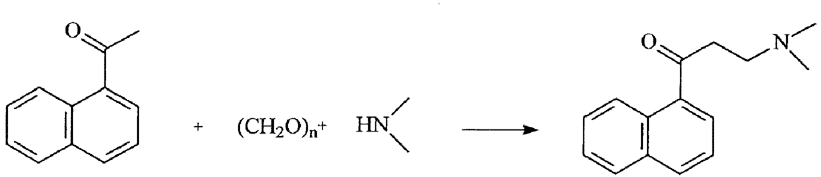

The mixture of 340 grams of the acetonaphthone, 56 grams of paraformaldehyde, and 200 grams of dimethylamine hydrochloride is boiled during 1.5 hours, then cooled down to room temperature, the sediment is filtered, treated with aqueous ammonia and diethyl ether. The organic layer is flushed with water, dried (MgS0₄), and filtered; the solvent is evaporated. Yield: 335 grams of the intermediate compound **IVa**.

### B. Obtaining claimed compounds

### Case 4

### 4-dimethylamino-2-(1-naphthyl)-1-phenyl-1-(2-chloro-6-bromoquinoline-3-yl)-butane-2-ol (IIa)

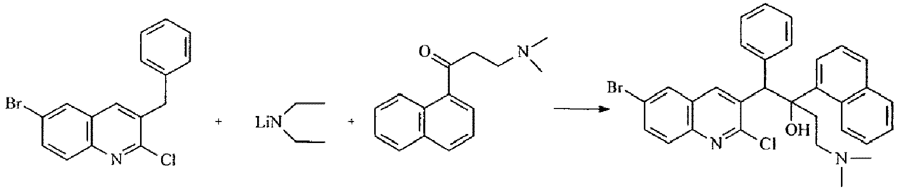

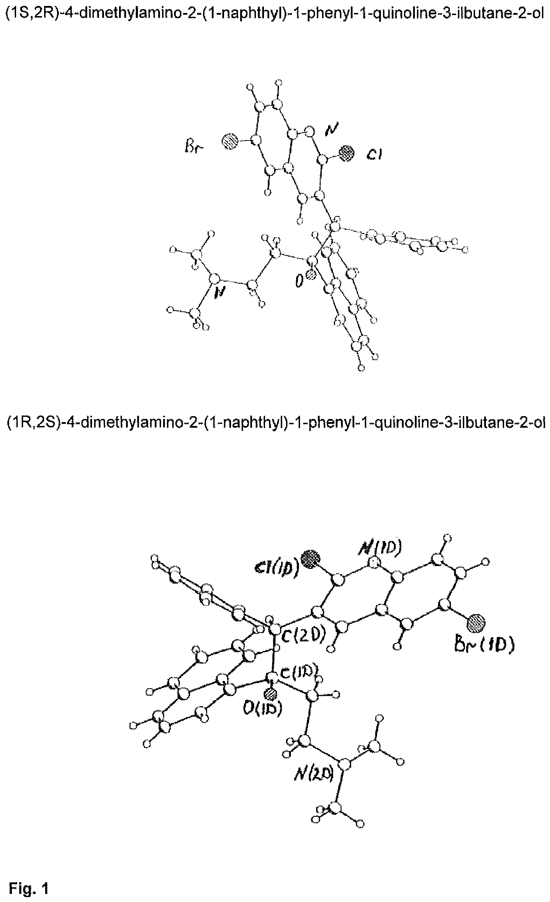

To the cooled down to -50°C solution of 190 grams of the intermediate naphthylketone **IVa** in 2 liters of dry tetrahydrofuran 50 grams of the diethylamide of lithium are added in an argon flow. The mixture is agitated during 1 hour, cooled down to -70°C; then 150 grams of the intermediate compound **IIIa** in 500 ml of tetrahydrofuran are added inside; the mixture is then cured during 2 hours at -70°C; then 50 ml of methanol are added inside. The mixture is warmed up to the room temperature, then 150 ml of 5 M solution of hydrogen chloride in isopropyl alcohol are added. The sediment is filtered and recrystallized from isopropyl alcohol. Yield: 67 grams. The compound **IIa** is obtained in the form of mixture of the optical isomers with the diastereoisomeric ratio (dr) close to 1:1. The diastereoselectivity of the process may be changed by using the appropriate ligands, solvents, and metalizing agents. Further on, the pairs (*RR*+*SS*) and (*RS*+*SR*) can be separated into enantiomericly clean products using the traditional methods, such as crystallization of the relevant diastereoisomeric salts from an appropriate solvent, or the chromatography. We illustrate the possibilities to separate the claimed compounds into individual stereoisomers by the case of the data of the X-ray crystallographic analysis of the two stereoisomers (1*S*,2*R*) and (1*R*,2*S*) of the compound **IIa**, obtained by separating the mixture of the isomers by one of the known methods (see Fig. 1).

### Case 5

The other derivatives **IIa-e** were obtained and separated in a similar way. Table 1 shows the characteristics of the obtained compounds.

**Table 1**

| | | | | | | | | | |
|---|---|---|---|---|---|---|---|---|---|
| 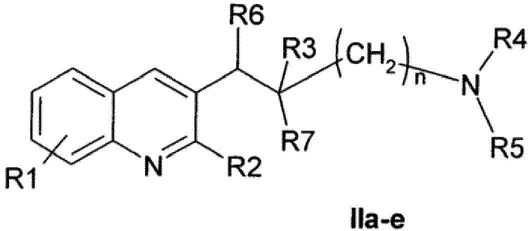 | | | | | | | | | |

| *Compound* | *R¹* | *R*² | *R³* | *R⁴* | *R⁵* | *R⁶* | *R⁷* | *n* | *[M+H]*⁺* |
|---|---|---|---|---|---|---|---|---|---|
| 1a | 6-Br | Cl | OH | Me | Me | Ph | 2-Nph | 2 | 561 |
| 1b | 6-Br | Cl | OH | Me | Me | *p*-C₆H₄-OMe | 2-Nph | 2 | 591 |
| 1c | 5-Me | Cl | OH | Me | Me | Ph | Ph | 2 | 496 |
| 1d | 6-Br | Cl | OH | Me | Me | Ph | Ph | 2 | 511 |
| 1e | 6-Br | Cl | OH | Me | Me | Ph | 2-Nph | 1 | 547 |

| | | | | | | | | | |
|---|---|---|---|---|---|---|---|---|---|
| *Main peak in the electrospray mass spectrum (ESI-MS) with positive ionization [ed. R. B. Cole Electrospray Ionization Mass Spectrometry, Wiley. - New York, 1997]. | | | | | | | | | |

### B. Brief description of antimycobacterial activity of obtained compounds

The obtained compounds of the general formula **II** have demonstrated a visible activity in relation to the *Mycobacterium tuberculosis* in the *in vitro* experiments. The most active representative of the line is the **IIa** one that has demonstrated a lower or commensurable minimal inhibitory concentration (MIC) than the comparator preparations rifampicine and isoniazid in the experiment on three strains of tuberculosis.

### 1. Determination of MIC100 (µg/ml) in agarized Dubos broth

The method of determination of the mycobacteria growth is based upon the use of the nitrate reductase activity of mycobacteria as criterion of their growth in the dense media containing KNO₃. The positive nitratreductase activity tuberculosis mycobacteria cultures are characterized by the capacity to reduce the nitrates into the nitrites. To indicate the potassium nitrate breakdown, the standard Griess reagent in form of the 7.5% aqueous solution is used. When the Griess reagent is added by drops to the mycobacteria medium it interacts with the formed nitrites and the medium surface becomes pink-red colored. Thus, the use of the biochemical reaction with the Griess reagent allows fixing the tuberculosis mycobacteria multiplication in short term (8 to 12 days) without visible growth of the culture.

The agarized Dubos broth was prepared in accordance with the recommendations of the manufacturer (Difco, USA) by adding the KNO₃ to the saline basis. After autoclaving of the saline basis in distilled water, the flask with the medium were cooled down to 52-54°C; then 5% sterile bovine serum albumin and sodium oleate were added. Then the agar was distributed into sterile test tubes, then, maintaining the 46-47°C temperature to prevent the solidification, the dilutions of the preparations # 1 and # 2 under study, and of the control preparations rifampicine (R) and isoniazide (H) were prepared. To study the bacteriostatic activity the following concentrations were used for all the preparations: 100 µg/ml; 50 µg/ml; 25 µg/ml; 12.5 µg/ml; 6.25 µg/ml; 3.125 µg/ml; 1.56 µg/ml; 0.78 µg/ml; and 0.39 µg/ml. The dilutions of the preparations under study and control ones and the dilutions of the solvent were distributed into the 24-socket plates; every concentration was duplicated. Thus, every plate contained the sockets with the agar with the panel of concentrations of the preparations in duplicates (experimental); the sockets with the agar without the preparations (positive control); and the sockets with the agar without the preparations that were not inoculated with bacteria (negative control or control of pullulation with nonspecific flora). The plates were incubated in the CO₂ incubator at 37°C, in presence of 5% of CO₂, during 10 days. 10 days after, the 7.5% aqueous solution of the Griess reagent was prepared and added to the plates sockets. The mycobacteria growth on the medium with preparations and in the control sockets was evaluated by the medium pink-red coloration.

**Table 2. Results of determination of the MIC100 (µg/ml) of the compound IIa for three strains of the M. tuberculosis.**

| Preparation | H37Rv | Poly MS-115 | Mono CN40 |
|---|---|---|---|
| Rifampicine | 0.2 | >200 | 0.2 |
| Isoniazid | 0.2 | 200 | 12.5 |
| **IIa** | 0.2 | 12.5 | 1.56 |

### 2. Determination of the concentrations of preparations inhibiting the growth of the mycobacteria in vitro, on the liquid Dubos broth, by variation of the optical density at 600 nm.

**Table 3**

| Values of the MIC100 for the compound **IIa** for different strains of the *M*. *tuberculosis* at the 11th day in the liquid Dubos broth | | | |
|---|---|---|---|
| | **H37Rv** | **CN-40** | **NIS-115** |
| **IIa** | 0.78 | 0.78 | 3.125 |
| Isoniazide | 0.1 | 6.25 | 50 |
| Rifampicine | 0.1 | 0.1 | >50 |

**Table 4**

| Values of the MIC100 for the compound **IIa** for different strains of the *M*. *Tuberculosis* at the 18th day in the liquid Dubos broth | | | |
|---|---|---|---|
| | **H37Rv** | **CN-40** | **MS-115** |
| **IIa** | 3.125 | 3.125 | >50 |
| Isoniazide | 0.1-0.4 | 6.25 and above | >50 |
| Rifampicine | 0.1 | 0.1 | >50 |

As Tables 2-4 show, the compound **IIa**, a typical representative of the group of aminoquinolines claimed in the general formula **II** demonstrates a higher efficacy in relation to the resistant strains of the *M*. *Tuberculosis,* than the comparator preparations, such as isoniazide and rifampicine.

## Claims

1. Compounds of the general formula II 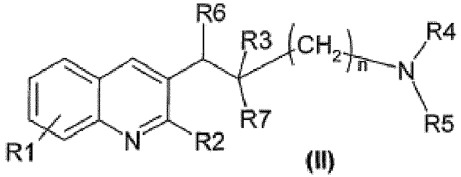 where
R1 means hydrogen, halogen, haloalkyl, dihaloalkyl, trihaloalkyl, cyano,
R2 means halogen,
R3 means hydroxy, mercapto, alkylthio, [amino(imino)methyl]thio, alkyldithio, difluormethyloxy,
R4, R5 mean, independently, hydrogen, alkyl, benzyl; or together, including an atom of nitrogen, they mean morpholinyl, piperidinyl, pyrrolidinyl, imidazolyl, triazinyl, tetrahydroazepinyl, piperazinyl, N'-alkylpiperazinyl, homopiperazinyl, N'-alkylhomopiperazinyl, pyrrolidinyl,
R6 means aryl, heteroaryl,
R7 means aryl, heteroaryl,
n= 1-4,
and their stereoisomers or mixes in any proportion, pharmaceutically acceptable acidic or basic additive salts, including the solvates.

2. Compounds claimed in claim 1, where R3 is a hydroxy group.

3. Process of production of the compounds claimed in claim 1, wherein the 2-arylmethylquinolines interact with the appropriate arylketones under the effect of a metalizing agent.

4. Process claimed in claim 3, wherein the diisopropylamide of lithium is used as the metalizing agent.

5. Compounds claimed in claim 1 for use in the treatment of infections induced by the mycobacteria.

6. Compounds claimed in claims 1 and 2 for use in the treatment of the tuberculosis.

7. Pharmaceutical composition, including the active substance, pharmaceutically acceptable carrier, and auxiliary additions, wherein the active substance is the compound claimed in claim 1 in the efficient amount.

## Patentansprüche

1. Verbindungen der allgemeinen Formel II, 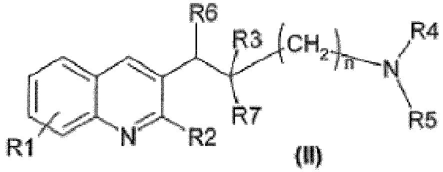 wobei
R1 Wasserstoff, Halogen, Haloalkyl, Dihaloalkyl, Trihaloalkyl, Cyano bedeutet,
R2 Halogen bedeutet
R3 Hydroxy, Mercapto, Alkylthio, [Amino(Imino)methyl]thio, Alkyldithio, Difluormethyloxy, bedeutet,
R4, R5 unabhängig voneinander Wasserstoff, Alkyl, Benzyl bedeuten; oder zusammen, umfassend ein Stickstoffatom, Morpholinyl, Piperidinyl, Pyrrolidiyl, Imidazolyl, Triazinyl, Tetrathydroazepinyl, Piperazinyl, N'-Alkylpiperazinyl, Homopiperazinyl, N'-Alkylhomopiperazinyl, Pyrrolidinyl bedeuten,
R6 Aryl, Heteroaryl bedeutet,
R7 Aryl, Heteroaryl bedeutet,
n= 1-4,
und ihre Stereoisomere oder Mischungen in jedem Verhältnis, pharmazeutisch akzeptable Salze durch Zugabe von sauren oder basischen Additiven, umfassend die Solvate.

2. Verbindung gemäß Anspruch 1, wobei R3 eine Hydroxygruppe ist.

3. Verfahren zur Herstellung der Verbindungen gemäß Anspruch 1, wobei die 2-Arylmethylquinoline mit den zweckmäßigen Arylketonen unter dem Einfluss eines metallisierenden Stoffs interagieren.

4. Verfahren gemäß Anspruch 3, wobei als metallisierender Stoff Lithiumdiisopropylamid verwendet wird.

5. Verbindungen gemäß Anspruch 1 zur Verwendung in der Behandlung von durch Mycobakterien induzierten Infektionen.

6. Verbindungen gemäß Anspruch 1 und 2 zur Verwendung in der Behandlung von Tuberkulose.

7. Pharmazeutische Zusammensetzung, umfassend die aktive Substanz, pharmazeutisch akzeptable Trägermaterialien, und weitere Hilfsstoffe, wobei die aktive Substanz eine Verbindung gemäß Anspruch 1 in ausreichender Menge ist.

## Revendications

1. Composés de formule générale II 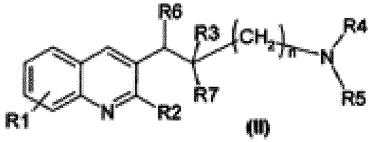 où :
R1 signifie un hydrogène, un halogène, un groupe halogénoalkyle, dihalogénoalkyle, trihalogénoalkyle, cyano,
R2 désigne un halogène,
R3 désigne un groupe hydroxy, mercapto, alkylthio, [amino(imino)méthyl]thio, alkyldithio, difluorométhyloxy,
R4, R5 désignent, indépendamment, un hydrogène, un groupe alkyle, benzyle ; ou conjointement, comprenant un atome d'azote, ils désignent un groupe morpholinyle, pipéridinyle, pyrrolidinyle, imidazolyle, triazinyle, tétrahydroazépinyle, pipérazinyle, N'-alkylpipérazinyle, homopipérazinyle, N'-alkylhomopipérazinyle, pyrrolidinyle,
R6 désigne un groupe aryle, hétéroaryle,
R7 désigne un groupe aryle, hétéroaryle,
n = 1 à 4,
et leurs stéréoisomères ou mélanges en une quelconque proportion, les sels additifs acides ou basiques pharmaceutiquement acceptables, y compris les produits de solvatation.

2. Composés selon la revendication 1, où R3 est un groupe hydroxy.

3. Procédé de production des composés selon la revendication 1, dans lequel les 2-arylméthylquinoléines interagissent avec les arylcétones appropriées sous l'effet d'un agent métallisant.

4. Procédé selon la revendication 3, dans lequel le diisopropylamide de lithium est utilisé comme agent métallisant.

5. Composés selon la revendication 1, destinés à être utilisés dans le traitement d'infections induites par les mycobactéries.

6. Composés selon les revendications 1 et 2, destinés à être utilisés dans le traitement de la tuberculose.

7. Composition pharmaceutique, comprenant la substance active, un véhicule pharmaceutiquement acceptable et des additions auxiliaires, dans laquelle la substance active est le composé selon la revendication 1 en la quantité efficace.
